# EUROPEAN PATENT APPLICATION

(11) **EP 1 749 521 A1**
(43) Date of publication of application: **07.02.2007**
(21) Application number: 06253984.6
(22) Date of filing: 31.07.2006
(51) Int. Cl.: A61K 9/70, A61K 31/465

(54) **Production method of nicotine transdermal preparation**

(30) Priority: 01.08.2005 JP 2005223475
(71) Applicant: NITTO DENKO CORPORATION, Ibaraki-shi, Osaka 567-8680 (JP)
(72) Inventor: Ninomiya, Kazuhisa, Nitto Denko Corporation, Ibaraki-shi Osaka 567-8680 (JP); Satoda, Shiro, Nitto Denko Corporation, Ibaraki-shi Osaka 567-8680 (JP); Saito, Junichi, Nitto Denko Corporation, Ibaraki-shi Osaka 567-8680 (JP); Kuroda, Hidetoshi, Nitto Denko Corporation, Ibaraki-shi Osaka 567-8680 (JP)
(74) Representative: Duckworth, Timothy John

(57) **Abstract**

The present invention provides a process for producing a nicotine transdermal preparation, which includes a step of forming, on a support or release liner, an adhesive layer comprising an adhesive and a liquid ingredient compatible with the adhesive, and a step of impregnating the adhesive layer with nicotine by continuously applying nicotine to the adhesive layer. The production method of the present invention permits direct application of nicotine to the adhesive layer, which is not possible by conventional techniques, and the nicotine transdermal preparation provided by the method is associated with less physical skin irritation on peeling off and affords a good feeling during adhesion.

## Description

### TECHNICAL FIELD OF THE INVENTION

The present invention relates to a production method of a nicotine transdermal preparation to be adhered to the outer skin to allow transdermal absorption of nicotine into the body.

### BACKGROUND OF THE INVENTION

It is well known that nicotine contained in cigarettes is deeply involved in habitual smoking. As a method for reducing smoking, administration of nicotine in a form other than smoking into the living organism has been proposed to suppress habitual smoking, and various nicotine administration methods have been proposed with the growing antismoking mood in the world. These methods are called what is called a nicotine supplement therapy, which includes the following methods.

One of them is a method of administering nicotine contained in a chewing gum or drug lozenge into the body from the mouth cavity. According to this administration method, in fact, a large amount of nicotine is swallowed down with the saliva, by which the nicotine is mostly metabolized and cleared from the blood during passage through the liver as in the case of oral administration of general drugs, and a high effect cannot be expected. Moreover, since this method is a temporary administration method, frequent application is necessary and, since nicotine directly touches the inner wall of the mouth and esophagus, uncomfortable side effects such as bad taste, heartburn, nausea, hiccup and the like are caused.

There is a method wherein a nicotine-containing solution is placed in a plastic one time container or multiple-time use container, which is then inserted into a nostril for direct administration of the nicotine solution in the container from the nasal mucous membrane. However, this method is not preferable from a hygiene standpoint, since the container directly contacts the nasal mucous membrane. In addition, handling and management is difficult. Moreover, since only a temporary effect is expected as in the above method, frequent administration is necessary. In particular, this method is problematic since it includes insertion of the container into the nostril, which makes administration in front of others embarrassing, and the like.

In contrast to the above-mentioned two administration methods, various transdermal preparations have recently been developed, which aim at administering nicotine transdermally. Many patent applications directed to transdermal preparations have been filed, some of which have already been put to practice as preparations.

While general advantages of the transdermal preparation are already known widely, when applied to administration of nicotine, in particular, the preparation can resolve almost all the shortcomings of the above-mentioned two administration methods. The greatest merit of the preparation is considered to rest in the fact that the blood concentration can be maintained at a constant level for a long time after adhesion of the preparation, which decreases the trouble of administration.

However, the existing transdermal preparations of nicotine have the following problems.
For use of these nicotine transdermal preparations, a stop-smoking program has been set to quit smoking, and the program generally requires once-a-day adhesion for several weeks. The major side effects of this administration method include topical side effects such as itching, erythema and the like. To avoid skin irritation, therefore, package inserts of the preparations contain a written instruction to change the adhesion site every time of application. Furthermore, the skin irritation developed by peeling off of the preparation cannot be ignored, since the preparation needs to be exchanged every day. Accordingly, the development of a preparation causing less irritation has been desired.

As the adhesive currently used for the nicotine transdermal preparations, rubber adhesives represented by polyisobutylene (PIB) and styrene-isoprene-styrene block copolymer (SIS), and acrylic adhesives made of a copolymer of acrylic monomers can be mentioned. Of these, for PIB adhesive, a technique including mixing a high molecular weight component and a low molecular weight component to impart good adhesiveness to the human skin and cohesion (e.g., JP-B-3035346) is available. However, to achieve good skin adhesion, cohesion needs to be sacrificed somewhat and, when adhesiveness is preferentially considered, a problem occurs in that an adhesive flows out from the edge of a preparation during preservation due to the decreased cohesion, thus causing a cold flow (low temperature flow). The cold flow invites difficulty in taking out the preparation from the packaging material due to the attachment of adhesive in the packaging material. Particularly, since nicotine has a strong plasticizing action on the adhesive, the above-mentioned cold flow phenomenon remarkably expresses in a nicotine transdermal preparation. While normal rubber adhesives adhere well to the dry skin, since adhesives have low hydrophilicity, sweat is pooled in the interface between the skin and the adhesion surface during application, which may lift the adhesive and cause delamination, thus resulting in falling off during use. Furthermore, the sweat develops stuffiness to easily cause irritation, and a feeling during adhesion is not necessarily good.

On the other hand, a nicotine transdermal preparation containing an acrylic adhesive is associated with a problem of skin irritation caused by peeling off for exchange of the preparation. Moreover, since the preparation contains a non-woven fabric or paper inserted in its adhesive layer as an auxiliary material for applying liquid nicotine to the adhesive layer during production, the whole preparation is thick, physical irritation easily occurs during application due to the rough feeling of the preparation, and a feeling during adhesion is not necessarily good.

Nicotine is a highly volatile and highly toxic drug, where volatilization of nicotine has a risk of increasing the safety risk and the burden on the environment. Accordingly, various production methods of nicotine transdermal preparations in consideration of this point have been known.

JP-A-2002-531488 discloses a production method of a transdermal patch, which comprises using a hexane solvent having a low boiling point to prepare a coating solution for a silicone adhesive, and applying the solution at a low temperature to decrease the decomposition or loss of a liquid drug of nicotine and the like. While this method employs coating at a low temperature, it cannot completely eliminate the possibility of loss of nicotine, and may require charging of an increased amount and the like to achieve the desired amount of nicotine. In addition, since silicone adhesives are expensive, this method is disadvantageous from an economical aspect.

JP-A-11-502840 discloses a continuous production method of a pressure-sensitive skin adhesive sheet material containing a liquid, which comprises combining a coating medium containing the liquid and a polymer base layer, wherein nicotine is exemplified as a liquid drug to be contained. The liquid application in this case is characterized in that a polymer component is contained in the coating medium, where direct and uniform application of the nicotine liquid was tried, namely, at 0% content of the polymer, but failed. The description suggests difficulty in applying nicotine as it is to an adhesive layer even by those of ordinary skill in the art.

JP-B-2708391 discloses a production method of a skin permeability administration tool comprising an absorption substance such as a non-woven fabric impregnated with highly volatile nicotine, and adhesive layers sandwiching the substance. It is described that the main non-woven fabric layer to be used permits printing of nicotine, and does not function as a drug reservoir. According to this method, the preparation becomes thick due to the non-woven fabric sandwiched in the plaster, which may impair a soft feeling of the preparation and influence adhesion of the preparation. The preparation is economically disadvantageous in that the production cost becomes high.

JP-B-2763773 discloses a production method of a treating product, by introducing a depot containing an active substance such as nicotine and the like into a reservoir matrix of an adhesive and the like. In the example of this reference, the depot containing nicotine contains EUDRAGIT E 100. The depot is added to a non-woven fabric to give a plaster, where the non-woven fabric as an inactive auxiliary substance helps uniform dispersion of nicotine. This reference does not disclose a technique to apply nicotine without using an inactive auxiliary substance.

### DISCLOSURE OF THE INVENTION

The present invention aims at providing a production method of a nicotine transdermal preparation permitting direct application of nicotine to an adhesive layer, which is not attainable by a conventional technique, and a production method of a nicotine transdermal preparation with less physical skin irritation due to peeling off and a good feeling during adhesion.

The present inventors have found that the above-mentioned object can be achieved by using, as a plaster layer of a nicotine transdermal preparation, an adhesive layer containing a large amount of a liquid ingredient, which resulted in the completion of the present invention.

Accordingly, the present invention provides the following.
[1] A production method of a nicotine transdermal preparation, which comprises
   a step of forming, on a support, an adhesive layer containing an adhesive and a liquid ingredient compatible with the adhesive, and
   a step of impregnating the adhesive layer with nicotine by continuously applying nicotine to the adhesive layer.
[2] The method of the above-mentioned [1], wherein the weight ratio of the adhesive and the liquid ingredient compatible with the adhesive is 1:0.25-1:1.8.
[3] The method of the above-mentioned [1] or [2], wherein the adhesive layer is impregnated with nicotine at a rate of 0.3-6.7 mg/cm²•min.
[4] The method of any of the above-mentioned [1]-[3], wherein the adhesive layer is an acrylic adhesive layer and has been crosslinked.

### BRIEF DESCRIPTION OF THE DRAWINGS

Fig. 1A is a schematic view showing a preferable embodiment of the present invention.
Fig. 1B is a schematic view showing a preferable embodiment of the present invention.
Fig. 2 is a graph showing the relationship between the impregnation rate and liquid ingredient content obtained in Experimental Examples.
Fig. 3 is a graph showing the results of the skin permeability test (Flux) of the preparations of Examples 1-3 and control in Experimental Examples.
Fig. 4 is a graph showing the results of the skin permeability test (Flux) of the preparations of Examples 4-6 and control in Experimental Examples.
In the Figures, 1 is a nicotine supply tank, 2 is a measuring pump, 3 is a die, 4 is a backup roll, 5 is an adhesive layer and 6 is a nicotine supply line.

### EFFECT OF THE INVENTION

According to the present invention, a nicotine transdermal preparation superior in both fixedness during adhesion and a feeling during adhesion (a soft feeling), which shows reduced skin irritation during peeling off and extremely superior adhesive properties can be obtained. Since the nicotine absorption rate of the adhesive layer can be increased by the addition of a large amount of a liquid ingredient to an adhesive layer, a continuously uniform nicotine transdermal preparation can be produced by directly applying the nicotine to the adhesive layer without necessity to dissolve in a solvent and the like. Moreover, since the nicotine content and/or concentration can be freely controlled by adjusting the application amount and/or the thickness of the adhesive layer, a preparation having various release properties can be produced easily.

### BEST MODE FOR EMBODYING THE INVENTION

The production method of the nicotine transdermal preparation of the present invention includes a step of forming an adhesive layer containing an adhesive and a liquid ingredient compatible with the adhesive on a support, and a step of impregnating the adhesive layer with nicotine by continuously applying nicotine to the adhesive layer.

In the present invention, nicotine is directly applied to an adhesive layer. To sufficiently afford the effect of the present invention, nicotine is preferably applied without addition of, for example, an auxiliary material such as auxiliary substances (e.g., solvent, polymer and the like), absorptive materials (e.g., non-woven fabric and the like), and the like.

The nicotine to be used for the present invention is free base nicotine, which permits easy transdermal absorption and direct application since it is liquid at ambient temperature.

While the amount of nicotine to be impregnated in an adhesive layer can be appropriately determined depending on the administration object, it is generally in an amount of about 1-40 wt% of the adhesive layer. When it is not less than 1 wt%, nicotine in an amount sufficient to achieve the treatment effect can be easily released, and when it is not more than 40 wt%, a highly cost effective transdermal preparation can be obtained.

For a low level nicotine to be averagely released, nicotine is preferably contained in a proportion of 1-20 wt%, more preferably 5-20 wt%, of an adhesive layer.

For a high level nicotine to be released at the initial stage of application, nicotine is preferably contained in a proportion of 20-40 wt%, more preferably 20-35 wt%, of an adhesive layer.

Since nicotine shows almost the same level of viscosity as does water at ambient temperature, when directly applied to a normal adhesive layer, nicotine is repelled and the adhesive layer cannot be impregnated with nicotine. Since the adhesive layer cannot be immediately impregnated with nicotine, a nicotine-containing transdermal preparation cannot be produced efficiently. A large amount of a liquid ingredient contained in an adhesive layer according to the present invention allows for conventional application methods for liquids as appropriate.

In the present invention, moreover, drugs other than nicotine may be contained in an adhesive layer, as long as no adverse influence is exerted on the desired use and practice of the present invention. As such drugs, for example, Mecamylamine, pempidine and the like as nicotine antagonists can be mentioned.

The adhesive to be used in the present invention may be those generally used in the field of transdermal preparation, such as rubber adhesives, vinyl adhesives, acrylic adhesives and the like. An adhesive permitting a crosslinking treatment is preferable.

As the rubber adhesive, for example, adhesives containing silicone rubber, polyisoprene rubber, polyisobutylene rubber, styrene-butadiene rubber, styrene-isoprene-styrene block copolymer rubber, styrene-butadiene-styrene block copolymer rubber and the like as a main component can be mentioned.

As the vinyl adhesive, for example, adhesives containing polyvinyl alcohol, polyvinyl alkyl ether, polyvinyl acetate and the like as a main component can be mentioned.

While the acrylic adhesive is not particularly limited, since crosslinking treatment is easy, a copolymer wherein (meth)acrylic acid alkyl ester has been copolymerized as a main component is preferably used. As (meth)acrylic acid alkyl ester, those wherein the alkyl group is a linear, branched chain or cyclic alkyl group having 4 to 18 carbon atoms (e.g., butyl, pentyl, hexyl, heptyl, octyl, nonyl, decyl, undecyl, dodecyl, tridecyl, 2-ethylhexyl, cyclohexyl etc.) are preferable. Such (meth)acrylic acid alkyl esters can be used in a combination of one or more kinds thereof. Of these, monomers that lower the glass transition temperature are preferable to afford adhesiveness at ambient temperature, and (meth)acrylic acid alkyl esters wherein the alkyl group is a linear, branched chain or cyclic alkyl group having 4 to 8 carbon atoms (e.g., butyl, pentyl, hexyl, heptyl, octyl, 2-ethylhexyl, cyclohexyl etc., preferably butyl, 2-ethylhexyl, cyclohexyl, particularly preferably 2-ethylhexyl) are more preferable. As the (meth)acrylic acid alkyl esters wherein the alkyl group has 4-8 carbon atoms, butyl acrylate, 2-ethylhexyl acrylate, 2-ethylhexyl methacrylate, cyclohexyl acrylate and cyclohexyl methacrylate are preferable, and 2-ethylhexyl acrylate is most preferable.

As the second component to be copolymerized with the above monomer, a monomer having a functional group capable of being involved in a crosslinking reaction may be used. A vinyl monomer having hydroxy group or carboxyl group as a functional group is preferably used in the present application. As the monomer of the second component, specifically, hydroxyethyl (meth)acrylate (e.g., 2-hydroxyethyl acrylate), hydroxypropyl (meth)acrylate, (meth)acrylic acid, itaconic acid, maleic acid, methaconic acid, citraconic acid, glutaconic acid and the like can be mentioned. These second monomer components can be used in combination of one or more kinds thereof.

A tertiary monomer component other than the second monomer component may be copolymerized. It can be used for adjusting cohesion of the adhesive layer, and adjusting solubility or releasability of nicotine or combined drug. As the tertiary monomer component, for example, vinyl esters such as vinyl acetate, vinyl propionate and the like; vinyl ethers such as methyl vinyl ether, ethyl vinyl ether and the like; vinyl amides such as N-vinyl-2-pyrrolidone, N-vinyl caprolactam and the like; hydroxy group-containing monomers such as (meth)acrylic acid alkyl ester, hydroxypropyl (meth)acrylate, α-hydroxymethyl acrylate and the like; amido group-containing monomers such as (meth)acrylamide, dimethyl(meth)acrylamide and the like; alkoxy group-containing monomers such as (meth)acrylic acid methoxyethyl ester, (meth)acrylic acid ethoxyethyl ester and the like; vinyl monomers such as styrene, vinyl pyridine, vinyl imidazole, vinyl morpholine and the like; and the like can be mentioned. These tertiary monomer components can be used in combination of one or more kinds thereof.

The acrylic adhesive can be obtained by, though not limited to, copolymerization of (meth)acrylic acid alkyl ester and the second monomer at a weight ratio of (meth)acrylic acid alkyl ester:second monomer=40-99.9:0.1-10.

When the tertiary monomer component is contained as necessary, the acrylic adhesive can be copolymerized by, though not limited to, adding (meth)acrylic acid alkyl ester, the second monomer and the tertiary monomer at a weight ratio of (meth)acrylic acid alkyl ester:second monomer:tertiary monomer=40-99.9:0.1-10:0-50.

The polymerization reaction is not particularly limited and can be carried out according to a method known per se. For example, a method comprising reacting the above-mentioned monomer by addition of a polymerization initiator (e.g., benzoyl peroxide, azobisisobutyronitrile and the like) in a solvent (e.g., ethyl acetate and the like) at 50-70°C for 5-48 hr can be mentioned.

As the adhesive, silicone rubber and acrylic adhesive are preferable, since a crosslinking treatment can be easily performed using a crosslinking agent.

In the present invention, the adhesive layer contains a liquid ingredient compatible with the adhesive in the adhesive layer. Addition of the liquid ingredient aims at imparting a soft feeling by plasticizing the adhesive, and reducing pain and skin irritation caused by adhesion to the skin when peeling off the nicotine transdermal preparation from the skin, and enabling direct coating of nicotine to the adhesive layer. Therefore, the liquid ingredient is not particularly limited as long as it has a plasticizing action and allows the present invention to be practiced, and one improving an absorption rate to the adhesive layer of the free base nicotine can be used. For combination with other drugs, one having an absorption promoting action can be used to improve the transdermal absorbability. As the liquid ingredient, for example, fats and oils such as olive oil, castor oil, squalene, lanoline and the like; organic solvents such as dimethyldecyl sulfoxide, methyloctyl sulfoxide, dimethyl sulfoxide, dimethylformamide, dimethylacetamide, dimethyllaurylamide, methylpyrrolidone, dodecylpyrrolidone and the like; liquid surfactants; diisopropyl adipate, phthalic acid (di)ester (e.g., diisononyl phthalate, di(2-ethylhexyl) phthalate and the like), plasticizers such as diethyl sebacate and the like; hydrocarbons such as liquid paraffin; fatty acid esters such as fatty acid alkyl ester (e.g., alcohol wherein the alkyl moiety is linear, branched chain or cyclic alkyl having 1 to 13 carbon atoms, ester with saturated or unsaturated fatty acid having 8 to 18 carbon atoms and the like, specifically, ethyl oleate, isopropyl palmitate, octyl palmitate, isopropyl myristate, isotridecyl myristate, ethyl laurate and the like), glycerol fatty acid ester (e.g., ester of glycerol and saturated or unsaturated fatty acid having 8 to 16 carbon atoms and the like, specifically, caprylic• capric triglyceride and the like), propylene glycol fatty acid ester (e.g., ester of propylene glycol and saturated or unsaturated fatty acid having 8 to 16 carbon atoms, and the like, specifically, propylene glycol dicaprylate and the like), pyrrolidonecarboxylic acid alkyl ester and the like; aliphatic dicarboxylic acid alkyl ester (e.g., ester of alcohol wherein the alkyl moiety is a linear, branched chain or cyclic alkyl having 1 to 4 carbon atoms and saturated or unsaturated aliphatic dicarboxylic acid having 6 to 16 carbon atoms, and the like, specifically, diisopropyl adipate, diethyl sebacate and the like); higher alcohol such as octyldodecanol; silicone oil; ethoxylated stearyl alcohol and the like can be mentioned. Of these, one or more kinds are used in combination. Of the above-mentioned, fatty acid alkyl ester and glycerol fatty acid ester are preferable. To be specific, isopropyl myristate, isopropyl palmitate and glycerol fatty acid ester are preferable, and of the glycerol fatty acid ester, caprylic•capric triglyceride is particularly preferable.

It is preferable to adjust the impregnation rate of an adhesive layer with nicotine to within the range of 0.3-6.7 mg/cm2·min by appropriately controlling the kind and amount of the liquid ingredient. When the impregnation rate is not less than 0.3 mg/cm²·min, an adhesive layer can be efficiently impregnated with a given amount of nicotine during the application step, and the nicotine content shows only a small dispersion. When the impregnation rate is not more than 6.7 mg/cm²·min, the ratio of the liquid ingredient in the adhesive layer is within a preferable range, adhesive properties such as adhesive force, cohesion, tack and the like are well balanced, and peeling off or adhesive residue does not occur easily.

The more preferable range of the impregnation rate is 0.5-5.0 mg/cm²•min, and the most preferable range is within the range of 0.8-3.8 mg/cm²·min. The weight ratio of the adhesive and the liquid ingredient in the adhesive layer to achieve these ranges is 1:0.25-1:1.8, preferably 1:0.4-1:1.6 from the aspect of skin irritation. In other words, the liquid ingredient is preferably contained in a greater amount.

Particularly, it is preferable to use fatty acid alkyl esters, particularly isopropyl myristate, glycerol fatty acid esters, particularly caprylic acid•capric triglyceride and the like as a liquid ingredient, and set the mixing ratio of the adhesive and the liquid ingredient in the adhesive layer for the above-mentioned range. Particularly, from the aspect of good balance between the transdermal absorbability and adhesion, a system coexisting a fatty acid alkyl ester and a glycerol fatty acid ester is preferable. To easily realize the above-mentioned mixing ratio of the liquid ingredient, a crosslinked acrylic adhesive is preferable.

The thickness of the adhesive layer is preferably 40-240 µm, more preferably 60-240 µm, and from the skin adhesion and transdermal absorbability of nicotine, it is preferably 50-200 µm, more preferably 100-200 µm.

In the present invention, the adhesive layer is preferably crosslinked to provide a suitable cohesion when applied to the skin of human and the like. As the crosslinking treatment, for example, a chemical crosslinking treatment using a crosslinking agent, such as an isocyanate compound (e.g., CORONATE HL (product name, manufactured by Nippon Polyurethane Industry Co., Ltd.) and the like), a metal chelate compound (e.g., metal chelate compound made of titanium, zirconium, zinc or aluminum, specifically aluminum ethylacetoacetate-diisopropylate (e.g., ALCH, product name, manufactured by Kawaken Fine Chemicals Co., Ltd.) and the like), organic peroxide, an epoxy compound, a melamine resin, metal alcoholate and the like, and a physical crosslinking treatment using UV, y ray, electron beam and the like can be mentioned. Of these, a chemical crosslinking treatment using a crosslinking agent, such as an isocyanate compound, metal alcoholate or a metal chelate compound consisting of titanium, zirconium, zinc, aluminum and the like is preferable from the aspects of reactivity and handling property. These crosslinking agents are free of a thickening phenomenon of the solution until application and drying, and are extremely superior in workability.

The amount of the crosslinking agent is about 0.01-5.0 parts by weight, per 100 parts by weight of the adhesive. Within this range, cohesion of the adhesive layer and skin adhesion are well balanced, and adhesive residue and skin irritation due to peeling off do not occur often.

The chemical crosslinking treatment can be performed according to a method known *per se.* Generally, after addition of a crosslinking agent, the mixture is heated to a temperature not lower than the crosslinking reaction temperature. The heating temperature and heating time can be appropriately determined according to the kind of the crosslinking agent. The heating temperature is generally about 50°C - 140°C, and the heating time is about 1 day to 1 week.

While the support is not particularly limited and any known support can be used, nicotine contained in the adhesive layer is preferably not lost from the back through the support to cause low content. Accordingly, the support is preferably made from a material impermeable to nicotine. Specifically, a single film of polyester, nylon, saran, polyethylene, polypropylene, ethylene-vinyl acetate copolymer, polyvinyl chloride, ethylene-ethyl acrylate copolymer, polytetrafluoroethylene, metal foil, polyethylene terephthalate and the like, a laminate film wherein one or more kinds thereof are laminated and the like can be used. To improve adhesion (anchor property) between the support and the adhesive layer, the support is, from among these, preferably a laminate sheet of a non-porous sheet made from the above-mentioned material and the following porous sheet, and the adhesive layer is preferably formed on the porous sheet side.

The porous sheet is not particularly limited as long as the anchor property between the support and the adhesive layer can be improved and, for example, paper, woven fabric, non-woven fabric (e.g., polyethylene terephthalate non-woven fabric and the like), a sheet obtained by a mechanical perforation treatment of the above-mentioned film (e.g., a single film of polyester, nylon, saran, polyethylene, polypropylene, ethylene-vinyl acetate copolymer, polyvinyl chloride, ethylene-ethyl acrylate copolymer, polytetrafluoroethylene, metal foil, polyethylene terephthalate and the like, a laminate film wherein one or more kinds thereof are laminated and the like), and the like can be mentioned. Particularly, paper, woven fabric, non-woven fabric (e.g., polyethylene terephthalate non-woven fabric and the like) are preferable. The thickness of the support is generally within the range of 10-500 µm, in consideration of the improvement of anchor property and flexibility of the whole preparation.

When a woven fabric or a non-woven fabric is used as a porous sheet, the amount of the fabric weight is preferably 5-50 g/m², more preferably 8-40 g/m², for the improvement of anchor property.

As the laminate sheet of a non-porous sheet and a porous sheet, a laminate sheet of a polyethylene terephthalate film and a polyethylene terephthalate non-woven fabric, and the like can be mentioned.

The nicotine transdermal preparation of the present invention is preferably protected until immediately before adhesion to the skin by covering the adhesive surface of the adhesive layer with a release liner. When in use, the liner is peeled off to expose the adhesive surface, which is then adhered to the adhesion site to allow administration of nicotine.

In the present invention, the adhesive layer may be formed on a release liner. In this case, an adhesive layer may be formed on a release liner, the adhesive layer is impregnated with nicotine, and a support may be adhered to the surface of the adhesive layer, which is opposite to the release liner.

The release liner is not particularly limited, and those generally used for transdermal preparations can be used. For example, a polyethylene terephthalate film peel treated with known peel treatment agents (e.g., long chain alkyl group-containing polymer, silicone polymer, fluorine polymer and the like) and the like can be mentioned. The thickness of the release liner is generally 25-500 µm.

As a preferable embodiment of the production method of the nicotine transdermal preparation of the present invention, the following methods can be mentioned.

A mixed solution of an adhesive and a liquid ingredient, and where necessary, a crosslinking agent is thoroughly stirred, the solution is applied to a support or a release liner, and dried. Then, a release liner or a support is adhered, and where necessary, a crosslinking treatment such as heating and the like is applied. Then, the release liner is peeled off, nicotine is directly applied onto the adhesive layer, and the release liner is adhered appropriately. When adhering the release liner, it is also possible to form a different adhesive layer and laminate the layer on an adhesive layer containing nicotine. The different adhesive layer to be laminated may have the same composition as the adhesive layer containing nicotine or have a different composition.

As a method for directly applying nicotine to the adhesive, a printing technique particularly employed in the printing field can also be used. When the viscosity needs to be adjusted when applying a printing technique, an additive may be used appropriately to the extent that the transdermal absorbability and adhesive properties are not influenced.

Examples of the method for applying nicotine include a method using a gravure coater, flexo coater, calendar coater, spray coater, curtain coater, fountain coater, die coater or slit die coater, inkjet and the like.

These methods can be adapted to thin film coating that general requires precision, and when the content uniformity of a drug is required as in the present invention, such a coating method is advantageously employed. Moreover, since nicotine is used as it is as a coating solution at this time, a coating method, wherein even a coating solution having a low viscosity can afford a high precision coating, is preferable. Moreover, since nicotine is extremely toxic, a highly safe coating method to a manufacturer is desirable, and therefore, a closed coating method is desirable. From such aspect, a method using a die coater or an inkjet printer of piezo system is particularly preferable, because of superior coating precision and easiness of making a closed system. A printing method can easily perform pattern coating on the surface of an adhesive layer, and is economically advantageous.

In the present invention, the most preferable method to apply nicotine is a method using a die coater, as shown below.

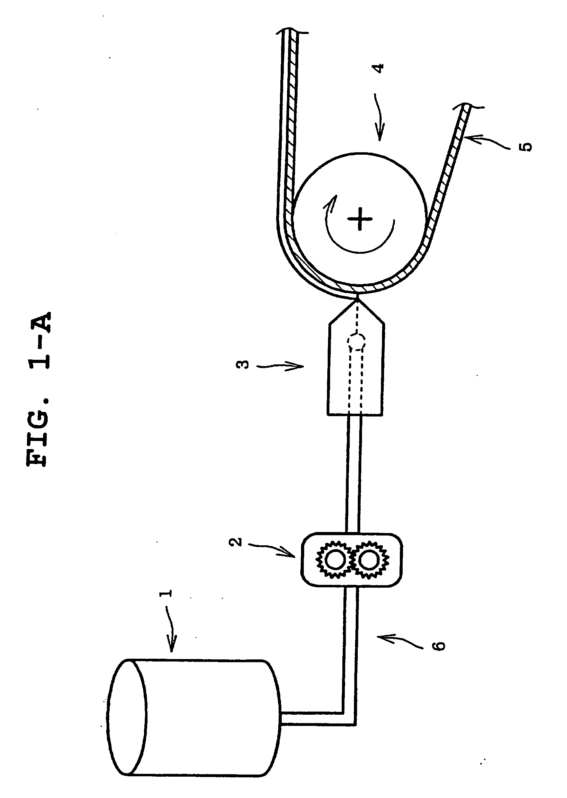

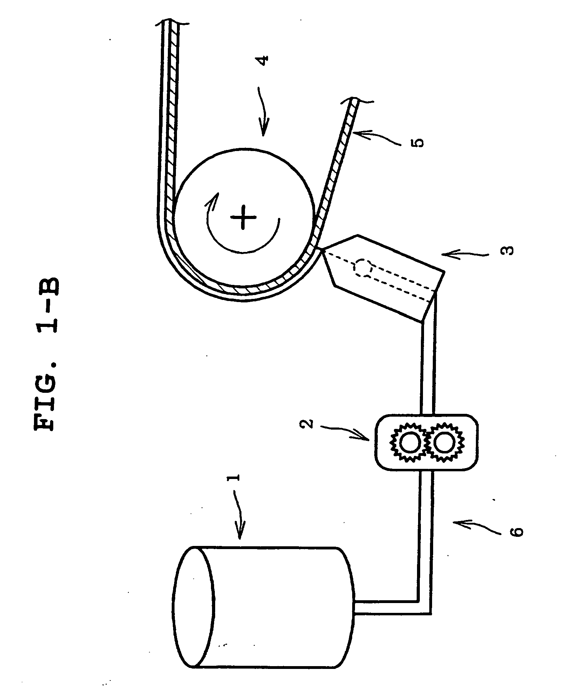

Fig. 1A and Fig. 1B show schematic views of one embodiment of die coating usable in the present invention.

Nicotine is supplied to die 3 from a nicotine supply tank 1 using a measuring pump 2. An adhesive layer 5 containing a liquid ingredient, which is supported by a support layer or a release liner, passes through a gap between a backup roll 4 and the die 3, and nicotine is uniformly applied to the adhesive layer 5 from the die 3.

As the die, for example, a curtain die, an ultra die, a lip die, a slot die and the like can be mentioned, with preference given to a slot die since it enables high precision coating with a low viscosity solution.

As the measuring pump, for example, a syringe pump, a gear pump, a mohno pump, a diaphragm pump and the like can be mentioned. In view of high precision and the like, a syringe pump is preferable, and a gear pump is also preferable.

The measurement precision of a pump is an important factor influencing the uniformity of nicotine application.

Not to mention the kind of measuring pump, the motor that drives the pump is also important, and a servo-type motor less susceptible to variation in the speed of rotation due to disturbance is preferably used.

In addition, the precision of the line speed of the adhesive layer 5 during application of nicotine is also important. The application amount of nicotine and application precision can be roughly determined based on the ratio of the speed of rotation and rotation precision of the measuring pump and the line speed alone. According to the production method of the present invention, since the nicotine absorption rate is sufficiently fast, the precision of the ratio of the speed of rotation of the measuring pump and the line speed can directly become the application precision.

As other factors influencing the uniformity of nicotine application, the pressure variation inside the nicotine supply line and the rheological characterization of nicotine inside the die can be mentioned. The pressure variation in the nicotine supply line may be caused by invasion of air bubbles into the supply line, besides the precision of the measurement pump. Thus, it is desirable to remove air bubbles inside the nicotine supply line. When a backup roll 4 is installed to keep the rotation axis horizontal so that the air bubbles can be easily removed, nicotine is desirably supplied from the intersection of the horizontal plane passing the rotation axis of the backup roll 4 and the outer circumference of the backup roll 4 or from the downstream in the rotation direction of the backup roll 4 (see Fig. 1A and Fig. 1B), and an air bubble trap (not shown) is desirably provided during the line. The pipe of a nicotine supply line 6 is desirably thin to facilitate removal of the air bubble. While the design of the diameter of the pipe varies depending on the supply amount of nicotine, when the nicotine supply amount is about 3 mL/min, the inner diameter of the pipe is desirably 2-4 mm. While the material of the pipe may be any as long as it is not corroded by nicotine, stainless is desirable since nicotine is poisonous. Even when the material of the pipe can be corroded by nicotine, a coating resistant to corrosion by nicotine can be applied to the inside of the pipe. It is preferable to use a Teflon (trade mark) pipe for confirmation of air bubbles inside the pipe.

The surface of the side to be applied (i.e., support or release liner) may have concaves and convexes of at least about ±5 µm.

In the present invention, nicotine is not dissolved in an auxiliary substance such as a solvent and the like but directly used as a coating solution. As a result, the coating solution has low viscosity, and the application.line speed can be raised. Consequently, the present invention is extremely advantageous for improving producibility and application precision.

The rheological characteristic of the coating solution in a die is also important for uniform application. Particularly, since the uniformity in the width direction of a wide die depends on the structure inside the die, a die sufficiently designed for nicotine application is preferably used.

The gap (shim) of the die for application of nicotine can be adjusted with a metal film or a plastic film inactive with nicotine. As a metal film inactive with nicotine, a stainless film, a zinc foil film, a titanium foil film and the like can be mentioned. As a plastic film inactive with nicotine, a polyethylene terephthalate film, a Teflon (trade mark) film, a cellulose acetate film, a polyvinyl chloride film, a polyethylene film, a polypropylene film, a polycarbonate film, a polyamide film and the like can be mentioned. The most preferable materials of shim include a polyethylene terephthalate film and a stainless film. While the thickness of the shim varies depending on the application thickness and speed of the application line, when the application thickness is 15-20 µm, it is preferably 20 µm - 100 µm.

Specific examples of the die system include slot die systems manufactured by LIBERTY, US and CLOEREN, US. However, the die system usable in the present invention is not limited to these. In addition, slot die systems manufactured by Chugai Ro Co., Ltd. and TORAY Engineering Co., Ltd., including measuring pumps, and the like can also be used preferably.

As mentioned above, since the application precision is determined based solely on the ratio of the speed of rotation of the measuring pump and the line speed in the nicotine application of the present invention, a device to control electric signals between the measuring pump and the line speed, and feedback the speed of rotation may be set, which is preferably designed to automatically increase the speed of rotation of the pump at a constant ratio as the line speed is increased.

Moreover, since the coating solution is poisonous, it is desirable to install a mechanism that automatically washes the head, inside, pipe and tank of the die. In addition, a safety cover may be set on a nicotine exposure part, or a ventilation device may be set in a workroom, to prepare for the occurrence of evaporation from the nicotine exposure surface.

In the present invention, nicotine is generally applied at room temperature. Inasmuch an indoor temperature change results in variation of specific gravity of nicotine, which in turn leads to the variation in the application amount, the temperature of nicotine to be applied is preferable maintained at a constant level. To maintain a constant temperature of nicotine, a device to maintain the temperatures of die, pipe and tank at constant levels may be equipped. When nicotine is applied at a high temperature, the infiltration speed of nicotine into an adhesive layer becomes high, though volatilization of nicotine places workers in danger.

Accordingly, for safety of workers, nicotine application at a low temperature is preferable, and the temperature of nicotine is maintained at 0-40°C, preferably 5-30°C, more preferably 10-25°C. The temperature change is preferably within ±2°C.

Since nicotine is hygroscopic, a long-term preservation in a highly humid place without humidity management should be preferably avoided. However, extremely low humidity may lead to inflammation and explosion of nicotine due to static spark. Therefore, nicotine is desirably applied at a place humidity-conditioned to a constant humidity (relative humidity 40-60%).

The shape and the size of the nicotine transdermal preparation produced by the method of the present invention are not particularly limited, and any shape and size can be employed according to the adhesion site and the like. The shape includes, for example, tape, sheet and the like. The size of the preparation is, for example, 5-30 cm²

The nicotine transdermal preparation produced by the method of the present invention can be used for a nicotine supplement therapy and the like of smokers (particularly those wishing to quit smoking), according to a stop-smoking program conventionally practiced or to be practiced in the future, which aims at suppressing habitual smoking.

While the dose of nicotine by the nicotine transdermal preparation produced by the method of the present invention varies depending on the age and body weight of the patients, severity of disease and the like, a transdermal preparation containing 5-120 mg of nicotine is generally adhered to the skin (5-30 cm²) of an adult once or so per 0.5 to 2 days.

### Examples

The present invention is explained in detail in the following by referring to Examples, which are not to be construed as limitative. Unless otherwise specified, part and % mean parts by weight and wt%, respectively, in the following.

### (Example 1)

Under a nitrogen atmosphere, 2-ethylhexyl acrylate (95 parts), acrylic acid (5 parts), ethyl acetate (100 parts) and benzoyl peroxide (0.2 part) were reacted in a separable flask equipped with a refluxing condenser, a stirrer, a thermometer, a dropping funnel and a nitrogen inlet tube at 60°C for 15 hr to give an adhesive solution.

The obtained adhesive solution was measured out in an amount corresponding to adhesive solid content of 49.93 parts and placed in a reaction container. Isopropyl myristate was added to the reaction container in 50 parts relative to the adhesive solid content, Coronate HL (manufactured by Nippon Polyurethane Industry Co., Ltd.) was added as a crosslinking agent in a proportion of 0.07 part (0.14% of the adhesive), and the mixture was thoroughly stirred.

The obtained solution was applied to a peel treated surface of a polyethylene terephthalate film release liner having the peel treated surface on one side to a thickness after drying of 240 µm, and dried at 60°C for 3 min, 80°C for 3 min and 95°C for 3 min to give an adhesive layer. The adhesive surface of the adhesive layer thus formed was adhered to the surface on a non-woven fabric side of a support prepared by laminating a 2 µm thick polyethylene terephthalate film on the polyethylene terephthalate non-woven fabric (fabric weight amount 12 g/m²) by extrusion forming to give a laminate. The laminate was tightly sealed, left standing at 60°C for 48 hr to form a crosslinked adhesive layer.

Thereafter, free base nicotine (i.e., free form of nicotine, without forming a salt) was applied to the adhesive surface of the adhesive layer with a die coater, while peeling off the release liner of the laminate to expose the adhesive surface. Then, a polyethylene terephthalate release liner was adhered to the nicotine-coated surface to give a nicotine transdermal preparation (width 100 mm, length 13 m).

### (Example 2)

Under a nitrogen atmosphere, 2-ethylhexyl acrylate (72 parts), N-vinyl-2-pyrrolidone (25 parts) and acrylic acid (3 parts) were charged in a flask, azobisisobutyronitrile (0.3 part) was added as a polymerization initiator, and polymerization was started. By adjusting the stirring rate and the outer bath temperature, and dropwise addition of ethyl acetate, the bath inner temperature was controlled to 58-62°C, and a polymerization reaction was carried out to give an adhesive solution (hereinafter to be also referred to as an adhesive solution A).

The above-mentioned adhesive solution was measured out in an amount corresponding to an adhesive solid content of 59.82 parts and placed in a reaction container. Coconad MT (manufactured by Kao Corporation, caprylic•capric triglyceride) was added to the reaction container in a proportion of 40 parts relative to the adhesive solid content, ALCH (manufactured by Kawaken Fine Chemicals Co., Ltd., aluminum ethylacetoacetate•diisopropylate) as a crosslinking agent was added in a proportion of 0.18 part (0.3% of the adhesive) and the mixture was thoroughly stirred.

The obtained solution was applied to a peel treated surface of a polyethylene terephthalate film release liner having the peel treated surface on one side to a thickness after drying of 120 µm, and dried at 70°C for 2 min and 90°C for 2 min to give an adhesive layer. The adhesive surface of the adhesive layer thus formed was adhered to the surface on a non-woven fabric side of a support prepared by laminating a 2 µm thick polyethylene terephthalate film on the polyethylene terephthalate non-woven fabric (fabric weight amount 12 g/m²) by extrusion forming to give a laminate. The laminate was tightly sealed, left standing at 60°C for 48 hr to form a crosslinked adhesive layer.

Thereafter, free base nicotine was applied to the adhesive surface of the adhesive layer with a die coater, while peeling off the release liner of the laminate to expose the adhesive surface. Then, a polyethylene terephthalate release liner was adhered to the nicotine-coated surface to give a nicotine transdermal preparation (width 100 mm, length 12 m).

### (Example 3)

2-Ethylhexyl acrylate/vinyl acetate/2-hydroxyethyl acrylate=78/16/6 (weight ratio, DURO-TAK2196, manufactured by National Starch & Chemical Company) was measured out in an amount corresponding to the adhesive solid content of 69.72 parts and placed in a reaction container. Coconad MT (manufactured by Kao Corporation) was added to the reaction container in a proportion of 30 parts relative to the adhesive solid content, ALCH (manufactured by Kawaken Fine Chemicals Co., Ltd.) as a crosslinking agent was added in a proportion of 0.28 part (0.4% of the adhesive) and the mixture was thoroughly stirred.

The obtained solution was applied to a peel treated surface of a polyethylene terephthalate film release liner having the peel treated surface on one side to a thickness after drying of 80 µm, and dried at 70°C for 2 min and 90°C for 2 min to give an adhesive layer. The adhesive surface of the adhesive layer thus formed was adhered to the surface on a non-woven fabric side of a support prepared by laminating a 2 µm thick polyethylene terephthalate film on the polyethylene terephthalate non-woven fabric (fabric weight amount 12 g/m²) by extrusion forming to give a laminate. The laminate was tightly sealed, left standing at 60°C for 48 hr to form a crosslinked adhesive layer.

Thereafter, free base nicotine was applied to the adhesive surface of the adhesive layer with a die coater, while peeling off the release liner of the laminate to expose the adhesive surface. Then, a polyethylene terephthalate release liner was adhered to the nicotine-coated surface to give a nicotine transdermal preparation (width 100 mm, length 14 m).

### (Examples 4, 5)

Under a nitrogen atmosphere, 2-ethylhexyl acrylate (72 parts), N-vinyl-2-pyrrolidone (25 parts), and acrylic acid (3 parts) were charged in a flask, and azobisisobutyronitrile (0.3 part) as a polymerization initiator was added to start polymerization. By adjusting the stirring rate and outer bath temperature, and dropwise addition of ethyl acetate, the inner bath temperature was adjusted to 58-62°C, and polymerization was carried out to give an adhesive solution.

The obtained adhesive solution was measured out in an amount corresponding to the adhesive solid content of 69.72 parts and placed in a reaction container. Isopropyl myristate was added to the reaction container in a proportion of 30 parts relative to the adhesive solid content, ALCH (manufactured by Kawaken Fine Chemicals Co., Ltd.) as a crosslinking agent was added in a proportion of 0.21 part (0.3% of the adhesive) and the mixture was thoroughly stirred.

The obtained solution was applied to a peel treated surface of a polyethylene terephthalate film release liner having the peel treated surface on one side to a thickness after drying of 40 µm (Example 4), 70 µm (Example 5), and dried at 70°C for 2 min and 90°C for 2 min to give an adhesive layer. The adhesive surface of the adhesive layer thus formed was adhered to the surface on a polyethylene terephthalate non-woven fabric side of a support prepared by laminating a 2 µm thick polyethylene terephthalate film on the polyethylene terephthalate non-woven fabric (fabric weight amount 12 g/m²) by extrusion forming to give a laminate. The obtained laminate was tightly sealed, left standing at 60°C for 48 hr to form a crosslinked adhesive layer.

Thereafter, free base nicotine was applied to the adhesive surface of the adhesive layer with a die coater, while peeling off the release liner of the laminate to expose the adhesive surface. Then, a polyethylene terephthalate release liner was adhered to the nicotine-coated surface to give a nicotine transdermal preparation (width 100 mm, length 10 m).

### (Example 6)

Under a nitrogen atmosphere, 2-ethylhexyl acrylate (72 parts), N-vinyl-2-pyrrolidone (25 parts), and acrylic acid (3 parts) were charged in a flask, and azobisisobutyronitrile (0.3 part) as a polymerization initiator was added to start polymerization. By adjusting the stirring rate and outer bath temperature, and dropwise addition of ethyl acetate, the inner bath temperature was adjusted to 58-62°C, and polymerization was carried out to give an adhesive solution.

The obtained adhesive solution was measured out in an amount corresponding to the adhesive solid content of 59.82 parts and placed in a reaction container. Isopropyl myristate was added to the reaction container in a proportion of 20 parts relative to the adhesive solid content, Coconad MT (manufactured by Kao Corporation) was added in a proportion of 20 parts relative to the adhesive solid content, ALCH (manufactured by Kawaken Fine Chemicals Co., Ltd.) as a crosslinking agent was added in a proportion of 0.18 part (0.3% of the adhesive) and the mixture was thoroughly stirred.

The obtained solution was applied to a peel treated surface of a polyethylene terephthalate film release liner having the peel treated surface on one side to a thickness after drying of 60 µm, and dried at 70°C for 2 min and 90°C for 2 min to give an adhesive layer. The adhesive surface of the adhesive layer thus formed was adhered to the surface on a polyethylene terephthalate non-woven fabric side of a support prepared by laminating a 2 µm thick polyethylene terephthalate film on the polyethylene terephthalate non-woven fabric (fabric weight amount 12 g/m²) by extrusion forming to give a laminate. The obtained laminate was tightly sealed, left standing at 60°C for 48 hr to form a crosslinked adhesive layer.

Thereafter, free base nicotine was applied to the adhesive surface of the adhesive layer with a die coater, while peeling off the release liner of the laminate to expose the adhesive surface. Then, a polyethylene terephthalate release liner was adhered to the nicotine-coated surface to give a nicotine transdermal preparation (width 100 mm, length 10 m).

### (Example 7)

Under a nitrogen atmosphere, 2-ethylhexyl acrylate (72 parts), N-vinyl-2-pyrrolidone (25 parts) and acrylic acid (3 parts) were charged in a flask, azobisisobutyronitrile (0.3 part) was added as a polymerization initiator, and polymerization was started. By adjusting the stirring rate and the outer bath temperature, and dropwise addition of ethyl acetate, the bath inner temperature was controlled to 58-62°C, and a polymerization reaction was carried out to give an adhesive solution.

The above-mentioned adhesive solution was measured out in an amount corresponding to an adhesive solid content of 34.88 parts and placed in a reaction container. Coconad MT (manufactured by Kao Corporation, caprylic•capric triglyceride) was added to the reaction container in a proportion of 65 parts relative to the adhesive solid content and Alumichelate A (manufactured by Kawaken Fine Chemicals Co., Ltd., aluminum tris(acetylacetonate)) was added as a crosslinking agent in a proportion of 0.12 part (0.35% of the adhesive) and the mixture was thoroughly stirred.

The obtained solution was applied to a peel treated surface of a polyester film release liner having the peel treated surface on one side to a thickness after drying of 70 µm, and dried at 100°C for 3 min to give an adhesive layer. The adhesive surface of the adhesive layer thus formed was adhered to the surface on a polyester non-woven fabric side of a support prepared by laminating a 2 µm thick polyethylene terephthalate film on the polyester non-woven fabric (fabric weight amount 12 g/m²) by extrusion forming to give a laminate. The laminate was tightly sealed, left standing at 60°C for 48 hr to form a crosslinked adhesive layer.

Thereafter, the release liner of the laminate was peeled off to expose the adhesive surface, a bar coater No. 9 (film thickness: about 20.6 µm) was set on a flexo printing coater (manufactured by RK Print Coat Instruments Ltd., product name: K-rocks proofer) and nicotine free base was uniformly applied on a stainless plate with the bar coater. Then, the adhesive surface of the crosslinked adhesive layer was adhered thereto to allow impregnation of the crosslinked adhesive layer with the nicotine free base (Sigma) at 1.65 mg/cm² Then, a polyester film release liner and the adhesive surface of the crosslinked adhesive layer of the above-mentioned laminate, which was applied with the nicotine free base were adhered to each other to give a nicotine transdermal preparation of Example 7.

### (Comparative Example 1)

The adhesive solution A (based on 100 parts of the adhesive solid content) obtained in Example 2 was directly (i.e., without liquid ingredient) applied to a peel treated surface of a polyethylene terephthalate film release liner having the peel treated surface on one side to a thickness after drying of 120 µm, and dried at 70°C for 2 min and 90°C for 2 min to give an adhesive layer. The adhesive surface of the adhesive layer thus formed was adhered to the surface on a non-woven fabric side of a support prepared by laminating a 2 µm thick polyethylene terephthalate film on the polyethylene terephthalate non-woven fabric (fabric weight amount 12 g/m²) by extrusion forming to give a laminate.

Thereafter, free base nicotine was applied to the adhesive surface of the adhesive layer with a die coater, while peeling off the release liner of the laminate. Then, a polyethylene terephthalate release liner was adhered to the nicotine-coated surface to give a nicotine transdermal preparation (width 100 mm, length 11 m).

### (Comparative Example 2)

An adhesive solution (74 parts:6 parts:20 parts isooctyl acrylate:acrylamide:vinyl acetate copolymer, solid content in 91 parts:9 parts ethyl acetate:methanol 22%, inherent viscosity =1.21 dl/g) was applied to a non-released surface of a silicone resin-coated polyethylene terephthalate release liner with an extrusion die. The die had a 20 mil (500 µm) shim. The coated release liner was dried in an oven at 150°F (65°C) for 1 min., 275°F (135°C) for 1 min., then 350°F (177°C) for 1 min. to give a web (width 7 inches (17.8 cm), 4000 line yards (3640 line meters).

Using direct gravure coating [gravure roll parameter:
pattern-triple helix; 45 line per inch (18 line per cm); volume factor -3.0×10⁻³ in³/in² (7.6×10⁻³ cm³/cm²), nicotine was uniformly applied as it was (i.e., 0% polymer content) but failed.

### (Experimental Examples)

The following evaluation was made with regard to the nicotine transdermal preparations obtained in Examples 1-7 and Comparative Example 1.

### Nicotine content

From the nicotine transdermal preparations obtained in Examples 1-6 and Comparative Example 1, 18-21 samples were obtained at 2 points (front and rear in Tables 1 - 8) at 25 mm from the both ends in the width direction as the center, and at 0.5 m intervals in the coating direction. For sampling, a 10 cm² square punching mold was used, and the nicotine transdermal preparation samples were punched out, extracted by shaking in methanol at 25°C for 120 min, and quantitated for the nicotine content of the extract by HPLC.
Three samples (sampled from the center portion of each sheet at three points of the beginning, middle and end in the coating direction) were taken from the obtained nicotine transdermal preparation of Example 7 (one sheet was cut into 9 cm×25 cm sheets, 10 sheets). For sampling, the samples of the nicotine transdermal preparation were obtained using a 10 cm² square punching mold, which were extracted with methanol at 25°C for 120 min with shaking, and the nicotine content of the extract was quantitated by HPLC.

### Measurement of nicotine impregnation rate

During coating the adhesive layers of Examples 1-6 and Comparative Example 1 with nicotine, the time from the coating to complete penetration of nicotine through the adhesive layers was measured as follows.

Nicotine on the adhesive was visually confirmed, and No. 1 filter paper defined in JIS P3801 was brought into contact with nicotine on the adhesive. The time point when impregnation of the filter paper with liquid nicotine stopped was taken as the point of complete impregnation of adhesive with nicotine. The distance from the nicotine discharge opening of the die to the impregnation point of adhesive with nicotine was measured. The impregnation rate was calculated by dividing the value by the coating rate.

For the nicotine content per square centimeters, the average of the nicotine content shown in the following Table was used. In the coating apparatus used for this Example, the distance from the nicotine discharge opening of the die to the liner adhesion site was 10 m. Therefore, the impregnation site of not less than 10 m could not be measured.
When coating the adhesive layer prepared in Example 7 with nicotine, the time necessary for complete impregnation of the adhesive layer with nicotine after the coating was measured as follows.
After uniform coating of the stainless plate with nicotine, the adhesive surface of the crosslinked adhesive layer was adhered thereto. The time necessary for nicotine to penetrate the crosslinked adhesive layer was set to 5 seconds to 1 minute at 5-second intervals, and then the obtained transdermal preparation was peeled off from the stainless plate. Nicotine remainder on the stainless plate was visually observed and the time when the residual nicotine disappeared was measured. The impregnated nicotine content was divided by the time to give the impregnation rate.

The results of the above-mentioned two tests are shown in Tables 1 - 8.

**Table 1**

| Test results of preparation of Example 1 | | |
|---|---|---|
| | nicotine content (mg/cm²) | |
| coating (m) | front | rear |
| 0.0 | 1.67 | 1.65 |
| 0.5 | 1.67 | 1.67 |
| 1.0 | 1.66 | 1.68 |
| 1.5 | 1.65 | 1.63 |
| 2.0 | 1.65 | 1.65 |
| 2.5 | 1.62 | 1.64 |
| 3.0 | 1.63 | 1.64 |
| 3.5 | 1.74 | 1.70 |
| 4.0 | 1.71 | 1.74 |
| 4.5 | 1.73 | 1.72 |
| 5.0 | 1.71 | 1.69 |
| 5.5 | 1.74 | 1.70 |
| 6.0 | 1.73 | 1.72 |
| 6.5 | 1.74 | 1.67 |
| 7.0 | 1.74 | 1.75 |
| 7.5 | 1.70 | 1.69 |
| 8.0 | 1.71 | 1.71 |
| 8.5 | 1.70 | 1.72 |
| 9.0 | 1.64 | 1.69 |
| 9.5 | 1.67 | 1.61 |
| 10.0 | 1.63 | 1.78 |
| average | 1.69 | |
| standard deviation | 0.042 | |
| relative standard deviation | 2.5% | |
| impregnation site | 0.8 m | |
| impregnation rate | 3.81 mg/cm²•min | |

**Table 2**

| Test results of preparation of Example 2 | | |
|---|---|---|
| | nicotine content (mg/cm²) | |
| coating (m) | front | rear |
| 0.0 | 1.60 | 1.58 |
| 0.5 | 1.62 | 1.54 |
| 1.0 | 1.68 | 1.62 |
| 1.5 | 1.64 | 1.59 |
| 2.0 | 1.62 | 1.58 |
| 2.5 | 1.56 | 1.52 |
| 3.0 | 1.69 | 1.58 |
| 3.5 | 1.70 | 1.66 |
| 4.0 | 1.65 | 1.67 |
| 4.5 | 1.63 | 1.62 |
| 5.0 | 1.56 | 1.59 |
| 5.5 | 1.59 | 1.57 |
| 6.0 | 1.56 | 1.59 |
| 6.5 | 1.56 | 1.58 |
| 7.0 | 1.58 | 1.60 |
| 7.5 | 1.58 | 1.63 |
| 8.0 | 1.59 | 1.68 |
| 8.5 | 1.56 | 1.66 |
| 9.0 | 1.58 | 1.70 |
| 9.5 | 1.58 | 1.64 |
| average | 1.61 | |
| standard deviation | 0.045 | |
| relative standard deviation | 2.8% | |
| impregnation site | 2.0 m | |
| impregnation rate | 1.45 mg/cm²·min | |

**Table 3**

| Test results of preparation of Example 3 | | |
|---|---|---|
| | nicotine content (mg/cm²) | |
| coating (m) | front | rear |
| 0.0 | 1.64 | 1.56 |
| 0.5 | 1.65 | 1.57 |
| 1.0 | 1.67 | 1.60 |
| 1.5 | 1.68 | 1.59 |
| 2.0 | 1.66 | 1.63 |
| 2.5 | 1.63 | 1.68 |
| 3.0 | 1.70 | 1.62 |
| 3.5 | 1.70 | 1.63 |
| 4.0 | 1.70 | 1.63 |
| 4.5 | 1.64 | 1.71 |
| 5.0 | 1.64 | 1.66 |
| 5.5 | 1.70 | 1.65 |
| 6.0 | 1.70 | 1.67 |
| 6.5 | 1.70 | 1.61 |
| 7.0 | 1.70 | 1.67 |
| 7.5 | 1.71 | 1.61 |
| 8.0 | 1.68 | 1.65 |
| 8.5 | 1.50 | 1.66 |
| 9.0 | 1.55 | 1.64 |
| 9.5 | 1.60 | 1.54 |
| 10.0 | 1.60 | 1.61 |
| average | 1.64 | |
| standard deviation | 0.051 | |
| relative standard deviation | 3.1% | |
| impregnation site | 3.5 m | |
| impregnation rate | 0.85 mg/cm²·min | |

**Table 4**

| Test results of preparation of Comparative Example 1 | | |
|---|---|---|
| | nicotine content (mg/cm²) | |
| coating (m) | front | rear |
| 0.0 | 1.70 | 1.64 |
| 0.5 | 1.59 | 1.66 |
| 1.0 | 1.56 | 1.51 |
| 1.5 | 1.74 | 1.81 |
| 2.0 | 1.54 | 1.48 |
| 2.5 | 1.60 | 1.59 |
| 3.0 | 1.62 | 1.62 |
| 3.5 | 1.66 | 1.63 |
| 4.0 | 1.73 | 1.75 |
| 4.5 | 1.54 | 1.61 |
| 5.0 | 1.47 | 1.65 |
| 5.5 | 1.46 | 1.65 |
| 6.0 | 1.42 | 1.62 |
| 6.5 | 1.45 | 1.63 |
| 7.0 | 1.45 | 1.64 |
| 7.5 | 1.39 | 1.61 |
| 8.0 | 1.36 | 1.59 |
| 8.5 | 1.41 | 1.57 |
| average | 1.58 | |
| standard deviation | 0.106 | |
| relative standard deviation | 6.7% | |
| impregnation site | not less than 10 m | |
| impregnation rate | not more than 0.28 mg/cm²·min | |

**Table 5**

| Test results of preparation of Example 4 | | |
|---|---|---|
| | Nicotine content (mg/cm²) | |
| coating (m) | front | rear |
| 0.0 | 1.93 | 2.07 |
| 0.5 | 2.06 | 2.09 |
| 1.0 | 2.01 | 2.05 |
| 1.5 | 1.98 | 2.05 |
| 2.0 | 2.03 | 2.04 |
| 2.5 | 2.02 | 2.06 |
| 3.0 | 2.01 | 2.04 |
| 3.5 | 2.02 | 2.04 |
| 4.0 | 2.01 | 2.07 |
| 4.5 | 2.01 | 2.06 |
| 5.0 | 2.00 | 2.07 |
| 5.5 | 1.98 | 2.07 |
| 6.0 | 2.00 | 2.07 |
| 6.5 | 1.99 | 2.08 |
| 7.0 | 1.99 | 2.08 |
| 7.5 | 2.01 | 2.08 |
| 8.0 | 2.01 | 2.09 |
| 8.5 | 2.02 | 2.06 |
| 9.0 | 2.01 | 2.07 |
| 9.5 | 2.05 | 2.10 |
| 10.0 | 2.03 | 2.09 |
| average | 2.04 | |
| standard deviation | 0.038 | |
| relative standard deviation | 2.0% | |
| impregnation site | 4.5m | |
| impregnation rate | 0.82 mg/cm²·min | |

**Table 6**

| Test results of preparation of Example 5 | | |
|---|---|---|
| | nicotine content(mg/cm²) | |
| coating (m) | front | rear |
| 0.0 | 1.77 | 1.79 |
| 0.5 | 1.76 | 1.78 |
| 1.0 | 1.77 | 1.77 |
| 1.5 | 1.76 | 1.78 |
| 2.0 | 1.77 | 1.77 |
| 2.5 | 1.77 | 1.78 |
| 3.0 | 1.75 | 1.78 |
| 3.5 | 1.77 | 1.76 |
| 4.0 | 1.76 | 1.75 |
| 4.5 | 1.76 | 1.77 |
| 5.0 | 1.76 | 1.76 |
| 5.5 | 1.76 | 1.77 |
| 6.0 | 1.75 | 1.76 |
| 6.5 | 1.77 | 1.76 |
| 7.0 | 1.76 | 1.77 |
| 7.5 | 1.72 | 1.75 |
| 8.0 | 1.75 | 1.74 |
| 8.5 | 1.75 | 1.75 |
| 9.0 | 1.75 | 1.75 |
| 9.5 | 1.75 | 1.75 |
| 10.0 | 1.76 | 1.76 |
| average | 1.76 | |
| standard deviation | 0.013 | |
| relative standard deviation | 1.0% | |
| impregnation site | 4.0 m | |
| impregnation rate | 0.79 mg/cm².min | |

**Table 7**

| Test results of preparation of Example 6 | | |
|---|---|---|
| | nicotine content(mg/cm²) | |
| coating (m) | front | rear |
| 0.0 | 2.12 | 2.04 |
| 0.5 | 2.15 | 2.00 |
| 1.0 | 2.14 | 2.01 |
| 1.5 | 2.13 | 2.02 |
| 2.0 | 2.13 | 1.99 |
| 2.5 | 2.11 | 2.00 |
| 3.0 | 2.13 | 2.00 |
| 3.5 | 2.12 | 2.00 |
| 4.0 | 2.10 | 2.03 |
| 4.5 | 2.09 | 2.07 |
| 5.0 | 2.13 | 2.12 |
| 5.5 | 2.11 | 2.11 |
| 6.0 | 2.12 | 2.11 |
| 6.5 | 2.14 | 2.09 |
| 7.0 | 2.15 | 2.09 |
| 7.5 | 2.15 | 2.07 |
| 8.0 | 2.14 | 2.05 |
| 8.5 | 2.15 | 2.03 |
| 9.0 | 2.14 | 1.99 |
| 9.5 | 2.14 | 1.98 |
| 10.0 | 2.19 | 2.09 |
| average | 2.09 | |
| standard deviation | 0.058 | |
| relative standard deviation | 3.0% | |
| impregnation site | 3.0 m | |
| impregnation rate | 1.25 mg/cm²·min | |

**Table 8**

| Test results of preparation of Example 7 | |
|---|---|
| sheet No. | nicotine content(mg/cm²) |
| 1 | 1.71 |
| | 1.63 |
| | 1.66 |
| 2 | 1.67 |
| | 1.64 |
| | 1.65 |
| 3 | 1.57 |
| | 1.70 |
| | 1.63 |
| 4 | 1.68 |
| | 1.55 |
| | 1.55 |
| 5 | 1.65 |
| | 1.71 |
| | 1.61 |
| 6 | 1.65 |
| | 1.65 |
| | 1.72 |
| 7 | 1.73 |
| | 1.69 |
| | 1.69 |
| 8 | 1.68 |
| | 1.72 |
| | 1.73 |
| 9 | 1.79 |
| | 1.83 |
| | 1.81 |
| 10 | 1.87 |
| | 1.65 |
| | 1.68 |
| average | 1.68 |
| standard deviation | 0.07 |
| relative standard deviation | 4.17 |
| impregnation site | 15 sec |
| impregnation rate | 6.72 mg/cm²·min |

The dispersion of the nicotine contents of the preparations of Examples 1-7 was 1.0-4.17% in relative standard deviation, and highly uniform preparations could be obtained. Moreover, according to the measurement of the impregnation rate, the preparations of the Examples showed very high nicotine impregnation rate, suggesting small coating non-uniformity.

In the nicotine coating in the preparation of Comparative Example 1, nicotine was hardly absorbed onto the coating surface of the adhesive layer but formed comparatively large water droplets on the coating surface, and the coating surface of the adhesive layer was observed not wet. The state of nicotine was almost the same until a release liner was adhered to the adhesive layer. It is assumed, therefore, that a decrease and dispersion in the nicotine content was caused by squeezing nicotine out during adhesion of the liner and the like. The impregnation rate of nicotine then was confirmed to be not more than 0.28 mg/cm²·min.

In contrast, in Comparative Example 2, which is based on the Examples of JP-A-11-502840, the experiment showed that the application of nicotine as it was onto an adhesive layer by a conventional method was extremely difficult.

From these experiments, it was shown that the production method of the nicotine transdermal preparation of the present invention could afford a highly uniform nicotine coating and could be highly industrialized.

### Relationship between impregnation rate and liquid ingredient

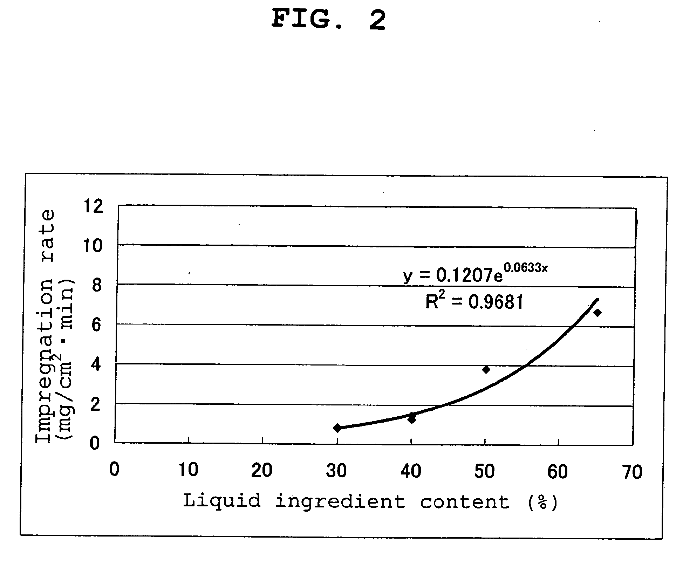

The relationship between the impregnation rate and the liquid ingredient content obtained in Examples 1-6 was plotted in a graph, and the relationship shown in Fig. 2 was obtained. While the relationship is assumed to vary depending on the kind of the liquid ingredient and adhesive, a greater amount of a liquid ingredient in an adhesive results in a higher impregnation rate.

### Evaluation of adhesiveness

### Adhesion:

The nicotine transdermal preparations of Examples 1-7 and Comparative Example 1 were cut into samples having a width of 24 mm, and the adhesion force of the samples was evaluated using a bakelite board as a deposit and a tensile tester (EZTest, manufactured by Shimadzu Corporation).

### Evaluation of pain upon peeling off:

The nicotine transdermal preparations of Examples 1-7 and Comparative Example 1 were formed into 10 cm² samples and adhered to the upper arm of 6 healthy volunteers for 24 hr. The pain upon peeling off of the preparations was evaluated according to the five level scores shown below.
1: not painful 2: only slightly painful
3: slightly painful 4: a little painful
5: very painful
The results are shown in Table 9.

**Table 9**

| Evaluation of adhesiveness | | | | | |
|---|---|---|---|---|---|
| | liquid ingredient | | adhesive force (N=3) N/24 mm | pain upon peeling off | falling off of sample in 6 volunteers during adhesion for 24 hr |
| | name | parts by weight relative to adhesive solid content | average | average score | |
| Ex. 1 | isopropyl myristate | 50 | 1.7 | 1.0 | 0/6 |
| Ex. 2 | Coconad MT | 40 | 3.3 | 1.3 | 0/6 |
| Ex. 3 | Coconad MT | 30 | 3.0 | 1.5 | 0/6 |
| Ex. 4 | isopropyl myristate | 30 | 1.9 | 1.6 | 0/6 |
| Ex. 5 | isopropyl myristate | 30 | 1.7 | 1.5 | 0/6 |
| Ex. 6 | isopropyl myristate | 20 | 1.1 | 1.4 | 0/6 |
| | Coconad MT | 20 | | | |
| Ex. 7 | Coconad MT | 65 | 0.85 | not measured | not measured |
| Comp. Ex. 1 | - | 0 | 5.5 | 4.7 | 0/6 |

### Evaluation of skin permeability

The drug permeability of the nicotine transdermal preparations was evaluated using the skin removed from hairless mouse and under the following conditions.
permeation apparatus: automated flow-through diffusion cell apparatus (manufactured by Vanguard International) sample area: 0.2826 cm²
receptor solution: phosphate buffer (pH=7.4), containing 0.02% sodium azide
flow: about 10 mL/4 hr/cell (pump rotation: 2.0 rpm)
sampling points: 1, 2, 3, 4, 5, 6, 9, 12, 15, 18, 21, 24 hr samples: nicotine transdermal preparations of Examples 1-3 (each n=3) and Examples 4-6 (each n=3)

As the comparison control of Examples 1-3, a commercially available nicotine transdermal preparation Nicotinell TTS (manufactured by Novartis) was used. As the comparison control of Examples 4-6, commercially available nicotine transdermal preparation NICODERM CQ CLEAR (manufactured by ALZA) was used.

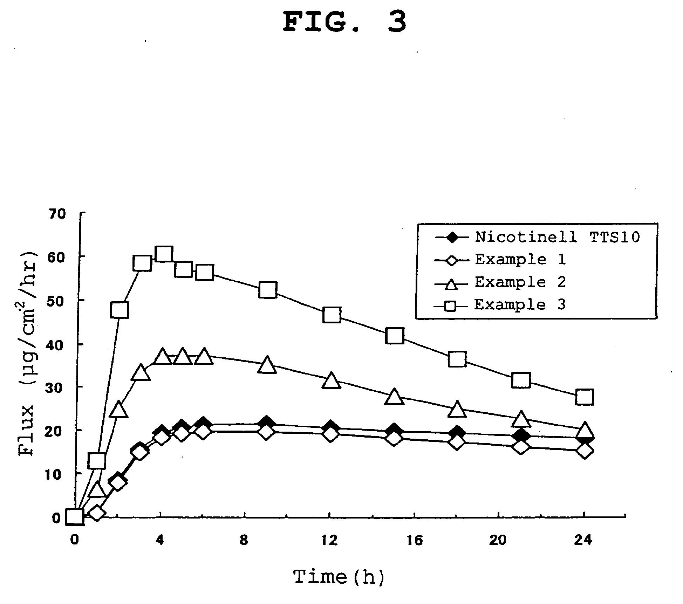

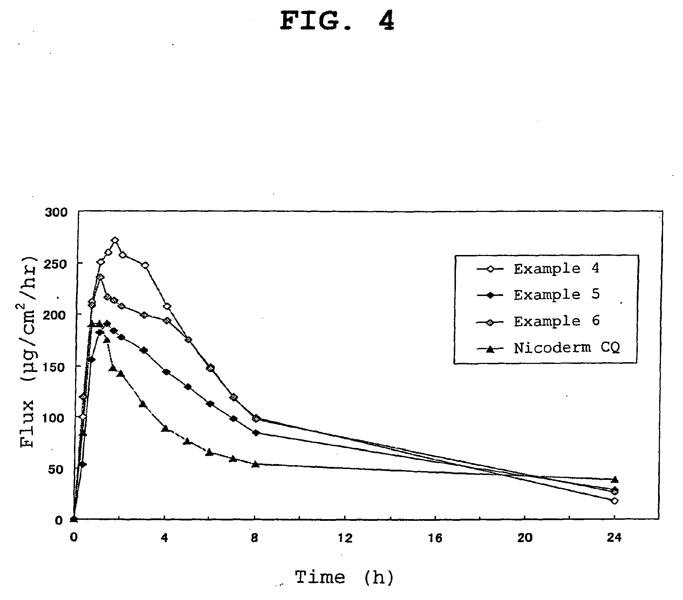

The content of nicotine flown into the receptor solution was quantitated by HPLC. The results are shown in Fig. 3 and Fig. 4.

As shown in the above, the preparations of Examples 1-6 showed extremely superior adhesion to the skin. They are preferable as a nicotine transdermal preparation to be adhered every day, since the pain upon peeling off is small and cause very small irritation. With no falling off during use, the preparations are highly economical. Moreover, the preparations of Examples 1-6 showed permeability of the same level as or not less than that of the existing nicotine transdermal preparations, as a result of the skin permeability test.

## Claims

1. A process for producing a nicotine transdermal preparation, which comprises
a step of forming, on a support or release liner, an adhesive layer comprising
an adhesive and a liquid ingredient compatible with the adhesive, and
a step of impregnating the adhesive layer with nicotine by continuously applying nicotine to the adhesive layer.

2. A process according to claim 1, wherein the weight ratio of the adhesive and the liquid ingredient compatible with the adhesive is 1:0.25-1:1.8.

3. A process according to claim 1 or claim 2, wherein the adhesive layer is impregnated with nicotine at a rate of 0.3-6.7 mg/cm²·min.

4. A process according to any one of claims 1 to 3, wherein the adhesive layer is an acrylic adhesive layer and is crosslinked.

5. A process according to any one of claims 1 to 4, comprising the following steps:
(i) forming, on a support or release liner, an adhesive layer comprising an adhesive and a liquid ingredient compatible with the adhesive;
(ii) adhering a release liner or support to the surface of the adhesive layer to form a structure comprising a support/adhesive layer/release liner;
(iii) peeling off the release liner and impregnating the adhesive layer with nicotine by continuously applying nicotine to the adhesive layer; and
(iv) adhering a release liner to the surface of the impregnated adhesive layer.

6. A process according to claim 5, wherein a crosslinking treatment is applied after step (ii) to crosslink the adhesive layer.

7. A process according to any one of claims 1 to 6, wherein the support comprises a polymeric material or metal foil impermeable to nicotine.

8. A process according to any one of claims 1 to 7, wherein the liquid ingredient comprises a fatty acid alkyl ester and/or a glycerol fatty acid ester.

9. A process according to any one of the preceding claims, wherein the nicotine is applied using a printing technique selected from a gravure coater, flexo coater, calendar coater, spray coater, curtain coater, fountain coater, die coater, slit die coater and inkjet.

10. A nicotine transdermal preparation obtainable by a process according to any one of the preceding claims.
